# EUROPEAN PATENT APPLICATION

(11) **EP 1 747 752 A1**
(43) Date of publication of application: **31.01.2007**
(21) Application number: 06253951.5
(22) Date of filing: 28.07.2006
(51) Int. Cl.: A61B 5/0205, A61B 5/0215, A61B 5/0452, A61N 1/365

(54) **Characterisation of patient's condition by evaluating electrical and mechanical properties of the heart**

(30) Priority: 28.07.2005 US 192538
(71) Applicant: PACESETTER, INC., Sylmar, CA 91392-9221 (US)
(72) Inventor: Gill, Jong, Valencia, CA 91355 (US); Min, Xiaoyi, Thousand Oaks CA 91362 (US)
(74) Representative: Rees, David Christopher

(57) **Abstract**

A device for evaluating the progression of a patient's condition, which in certain applications can include heart failure, on an ongoing manner which reduces the need for immediate attention from skilled clinicians or expensive diagnostic equipment. The device analyses relative timing between electrical and mechanical properties of the heart. Detection of an elongated delay between corresponding electrical and mechanical activity is interpreted as indicating a worsening heart failure condition. The analysis and data corresponding thereto can be stored for further analysis and/or telemetrically communicated to an external device. Therapy provided by the device can be altered based on the evaluation of the patient's condition.

## Description

The invention relates to the field of implantable medical devices and more particularly to devices and algorithms for automatically measuring and characterizing a patient's condition, for example in detecting the onset or status of a heart failure (HF) condition, by evaluating electrical and mechanical properties of the heart.

Heart failure (HF) refers broadly to a variety of health ailments characterized by a reduction in the mechanical ability of the heart to deliver an appropriate supply of blood. Heart failure can encompass an enlargement of the heart muscle, a degradation of the contractile properties of the heart, and/or a reduction in the synchrony in the cardiac contractions. Heart failure can also correspond to damage to or deterioration of heart valves and other structural conditions which reduce the cardiac output. Heart failure is also frequently found coincident with a variety of cardiac arrhythmias.

Heart failure can be of a varying degree of severity, ranging from the least severe where the HF condition may be detected upon clinical evaluation and wherein overt symptoms may only be noticed during strong physical exertion to the most severe conditions of HF, wherein the patient experiences severe symptoms even when fully resting. A variety of therapies are available to treat HF and the severity and progress of an HF condition is a valuable indicator for the patient's overall health status. Thus, it will be appreciated that being able to readily identify and characterize either the onset of an HF condition or the ongoing severity of an HF condition can provide a valuable diagnostic tool to a clinician to provide more effective therapy to the patient.

A variety of examinations and observations can be utilized by a clinician to evaluate the existence or progression of an HF condition. A physical examination and interview of the patient can reveal, for example, edema and/or weight gain caused by fluid accumulation, which is a frequent symptom of HF. Shortness of breath is also a common symptom of HF and an interview of the patient and examination can reveal the severity of and conditions under which the shortness of breath occurs. An examination can also reveal a third heart sound, frequently referred to as S3, as well as a sound of fluid in the lungs during inspiration (rales), either of which are common symptoms of HF. A clinician may also observe enlargement of the jugular vein in the neck region (jugular venous distention), enlargement of the liver (hepatomegaly), and this may be coupled with a hepatojugular reflex wherein an enlarged liver which is subjected to manual pressure forces more blood into the jugular veins, causing them to become even more enlarged.

Several diagnostic tests are also useful in diagnosing HF, including chest x-rays which can reveal pulmonary edema, an enlarged heart, and pleural effusion. Electrocardiograms (EKGs) are also useful for their ability to detect the presence of a heart attack, cardiac ischemia, abnormal heart rhythms, and/or an enlarged heart. Echocardiograms are also useful diagnostic tools which can determine the amount of blood ejected from the heart with each heartbeat, and more particularly, the proportion of blood ejected which is typically referred to as the ejection fraction. The ejection fraction is a useful way to quantitatively characterize the efficiency of the heart which is closely related to the presence or severity of a HF condition. For a normal healthy person, the ejection fraction typically is in the range from approximately 55 to 75%. A person suffering from HF would typically have a lower ejection fraction with a more depressed ejection fraction indicating a more severe HF condition. Echocardiograms can also diagnose particular causes of HF, including heart valve abnormalities, pericardial abnormalities, congenital heart disease, and/or an enlarged heart. Echocardiograms can also show if the contraction of the heart itseif is abnormal, such as in wall motion abnormalities.

While these clinical observations and diagnostic tests offer valuable information for diagnosing the progress of a heart failure condition, they suffer from the disadvantage of requiring the direct intervention of a highly trained clinician. The aforementioned patient observations require the training and judgment of a skilled clinician to accurately diagnose the patient observations. The aforementioned diagnostic tests, in addition to requiring the services of a skilled clinician also typically require that the tests take place in a clinical setting. Diagnostic equipment such as chest x-ray and echocardiogram machines are large, complex, and relatively expensive pieces of equipment which are neither portable nor economical for the dedicated service of a single patient. Thus, the aforementioned observations and diagnostic tests are not suitable for frequent ongoing diagnosis of a patient's condition but rather are more suitable to serve a large number of patients at scheduled clinical appointments.

Thus, it will be appreciated that the ability to more frequently evaluate a patient, such as for the progress of an HF condition, on an ongoing manner without requiring the immediate attention of a skilled clinician and expensive complex diagnostic equipment, could provide valuable diagnostic information to more accurately and timely track the patient's condition. Thus, there is an ongoing need for a system and method of evaluating a patient's condition in a portable relatively inexpensive manner which would facilitate evaluation of the condition on a frequent ongoing manner and more particularly in intervals between clinical evaluations.

According to the invention, there is provided an implantable medical device comprising: an electrical activity detector for detecting electrical activity of a patient's heart; and a mechanical activity detector for detecting mechanical activity of the heart; characterised by means for determining a delay between corresponding monuments of the electrical activity and of the mechanical output for a given cardiac cycle; and means for evaluating the patient's condition based at least partially on the delay.

According to another aspect of the invention, there is provided an implantable medical device comprising at least one implantable sensing electrode configured for measuring electrical activity of a patient's heart; characterised by: at least one implantable mechanical sensor configured to measure the mechanical activity of the heart; and a controller in communication with the sensing electrode and mechanical sensor, wherein the controller is operative to evaluate the relative timing between the electrical and mechanical activity and determine a health indicator based at least in part on the evaluation.

The invention contemplates a method of evaluating a patient's condition, the method comprising: detecting electrical activity of a patient's heart with an implantable device; detecting mechanical activity of the heart with the implantable device; determining a delay between corresponding monuments of the electrical activity and of the mechanical output for a given cardiac cycle; and evaluating the patient's condition based at least partially on the delay. Evaluating the patient's condition may comprise evaluating the efficacy of a previously instituted cardiac resynchronization therapy (CRT) regimen.

The method may further comprise providing therapy with the device according to at least one of a programmable atrioventricular (AV) delay and a programmable ventricular-ventricular (W) delay and programming at least one of the AV and VV delays of the device based at least in part on the evaluation of the patient's condition.

Detecting mechanical activity preferably comprises measuring a pressure of blood pumped from the heart, and evaluating the patient's condition preferably comprises evaluating a degree of heart failure.

The method may further comprise generating an alert based on the evaluation of the patient's condition and preferably also communicating results of the evaluation to an external device. Preferably, the implantable device is capable of delivering therapy to the patient and further comprising adjusting one or more therapy-related parameters of the device based at least in part on the evaluating.

The invention extends to an implantable cardiac stimulation device comprising: at least one lead adapted to be implanted within a patient, the at least one lead further adapted to provide therapeutic stimulation to the heart of the patient; at least one sensor that monitors a plurality of parameters indicative of activity of the heart wherein the plurality of parameters are related; and a controller that receives signals indicative of the plurality of parameters from the at least one sensor and further induces the delivery of therapeutic stimulation by the implantable lead to the heart, wherein the controller periodically records the plurality of related parameters received from the at least one sensor to create a record indicative of a correlation between electrical and mechanical activity of the heart.

Preferably, the at least one sensor receives electrical signals indicative of the activity of the heart as indicated by an intracardiac electrogram, and may comprise a mechanical sensor that detects mechanical activity of the heart. Preferably, the controller evaluates derived characteristics of the plurality of parameters and determines corresponding monuments wherein the derived characteristics comprise first derivatives with respect to time and wherein the controller utilizes the derivative monuments to correlate between the electrical and mechanical activity of the heart.

The device may further comprise a telemetry circuit adapted for communication with an external device such that the device can communicate the record indicative of the correlation between the electrical and mechanical activity of the heart externally.

Certain embodiments described herein evaluate relationships between mechanical activity and electrical activity of the heart to characterize detection and progression of an HF condition. Furthermore, this relationship can be used to evaluate the efficacy of Cardiac Resynchronization Therapy (CRT), AV/VV optimization, and/or left ventricular lead placement. More particular embodiments evaluate relative timing or delays between an observed electrical characteristic, for example, electro-chemical activity inducing a contraction and the corresponding mechanical activity, for example the contraction of the cardiac tissue. Embodiments evaluate this relative timing or delay between electrical and mechanical activity with evaluation made with respect to the metric that an increased delay between electrical activity and corresponding mechanical activity indicates onset or worsening of an HF condition depending on the magnitude of the delay. Thus, embodiments determine that an increased disassociation between the electrical and mechanical activities of the heart is indicative of a worsening condition, for example a worsening of HF.

Further embodiments provide a relatively inexpensive device which can be provided to a patient on a long-term basis, such as via implantation, which evaluates the relative coordination between the electrical and mechanical activity of the heart and is capable of determining a change in this relative timing, such as in elongation of the electro-mechanical delay and in certain embodiments stores this information for further evaluation by a clinician and/or provides the data telemetrically for further evaluation.

One embodiment comprises a method of evaluating a patient's condition with an implantable device, the method comprising monitoring electrical activity of the heart with an implantable device, monitoring mechanical output of the heart with the implantable device, determining reference monuments of both the monitored electrical activity and mechanical output, determining a delay between corresponding monuments of the electrical activity and of the mechanical output for a given cardiac cycle, and evaluating the patient's condition based at least partially on the delay.

Another embodiment comprises an implantable medical device comprising at least one implantable sensing electrode configured for measuring electrical activity of a patient's heart, at least one implantable mechanical sensor configured to measure the mechanical activity of the heart, and a controller in communication with both the sensing electrode and mechanical sensor wherein the controller evaluates the relative timing between the electrical and mechanical activity and determines a health indicia based at least in part on the evaluation.

These and other objects and advantages of the invention will become more apparent from the following description taken in conjunction with the accompanying drawings, in which:

**Fig. 1** is a simplified diagram illustrating an implantable stimulation device in electrical communication with at least three leads implanted into a patient's heart for delivering multi-chamber stimulation and shock therapy;

**Fig. 2** is a functional block diagram of a multi-chamber implantable stimulation device illustrating the basic elements of a stimulation device which can provide cardioversion, defibrillation and pacing stimulation in four chambers of the heart;

**Fig. 3A** is an example waveform of one embodiment of monitoring electrical activity of the heart;

**Fig. 3B** is an example waveform of one embodiment of monitoring mechanical output of the heart;

**Fig. 3C** is an example waveform of the rate of change of the electrical activity of the heart indicated in the waveform of **Fig. 3A;**

**Fig. 3D** is an example waveform of the rate of change of the mechanical output of the heart indicated in the waveform of **Fig. 3B;** and

**Fig. 4** is a flow chart of one embodiment of a method of evaluating a patient's condition, which can include evaluation of heart failure, based at least partially on monitoring of electrical activity and mechanical output of the heart.

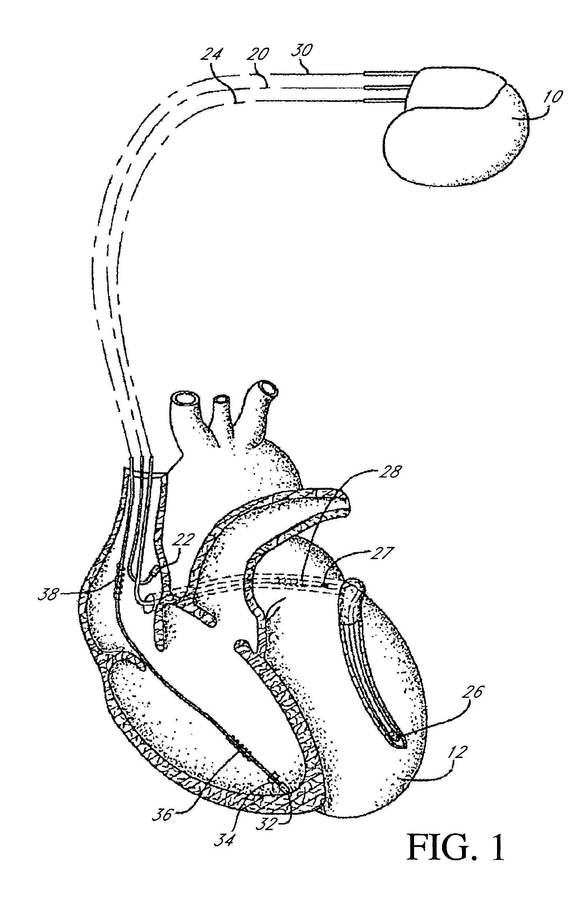

In one embodiment, as shown in **Fig. 1,** a device 10 comprising an implantable cardiac stimulation device 10 is in electrical communication with a patient's heart 12 by way of three leads, 20, 24 and 30, suitable for delivering multi-chamber stimulation and shock therapy. To sense atrial cardiac signals and to provide right atrial chamber stimulation therapy, the stimulation device 10 is coupled to an implantable right atrial lead 20 having at least an atrial tip electrode 22, which typically is implanted in the patient's right atrial appendage.

To sense left atrial and ventricular cardiac signals and to provide left chamber pacing therapy, the stimulation device 10 is coupled to a "coronary sinus" lead 24 designed for placement in the "coronary sinus region" via the coronary sinus ostium (OS) for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, an exemplary coronary sinus lead 24 is designed to receive atrial and ventricular cardiac signals and to deliver left ventricular pacing therapy using at least a left ventricular tip electrode 26, left atrial pacing therapy using at least a left atrial ring electrode 27, and shocking therapy using at least a left atrial coil electrode 28.

The stimulation device 10 is also shown in electrical communication with the patient's heart 12 by way of an implantable right ventricular lead 30 having, in this embodiment, a right ventricular tip electrode 32, a right ventricular ring electrode 34, a right ventricular (RV) coil electrode 36, and a superior vena cava (SVC) coil electrode 38. Typically, the right ventricular lead 30 is transvenously inserted into the heart 12 so as to place the right ventricular tip electrode 32 in the right ventricular apex so that the RV coil electrode will be positioned in the right ventricle and the SVC coil electrode 38 will be positioned in the superior vena cava. Accordingly, the right ventricular lead 30 is capable of receiving cardiac signals, and delivering stimulation in the form of pacing and shock therapy to the right ventricle.

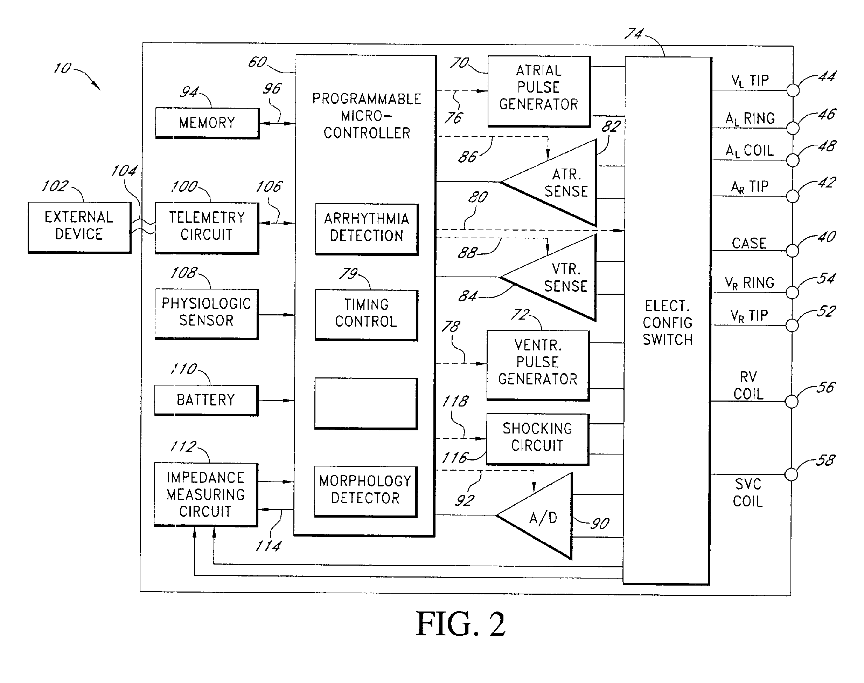

As illustrated in **Fig. 2**, a simplified block diagram is shown of the multi-chamber implantable stimulation device 10, which is capable of treating both fast and slow arrhythmias with stimulation therapy, including cardioversion, defibrillation, and pacing stimulation. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and pacing stimulation.

The housing 40 for the stimulation device 10, shown schematically in **Fig. 2**, is often referred to as the "can", "case" or "case electrode" and may be programmably selected to act as the return electrode for all "unipolar" modes. The housing 40 may further be used as a return electrode alone or in combination with one or more of the coil electrodes, 28, 36 and 38, for shocking purposes. The housing 40 further includes a connector (not shown) having a plurality of terminals, 42, 44, 46, 48, 52, 54, 56, and 58 (shown schematically and, for convenience, the names of the electrodes to which they are connected are shown next to the terminals). As such, to achieve right atrial sensing and pacing, the connector includes at least a right atrial tip terminal (A_{R} TIP) 42 adapted for connection to the atrial tip electrode 22.

To achieve left chamber sensing, pacing and shocking, the connector includes at least a left ventricular tip terminal (V_{L} TIP) 44, a left atrial ring terminal (A_{L} RING) 46, and a left atrial shocking terminal (A_{L} COIL) 48, which are adapted for connection to the left ventricular tip electrode 26, the left atrial ring electrode 27, and the left atrial coil electrode 28, respectively.

To support right chamber sensing, pacing and shocking, the connector further includes a right ventricular tip terminal (V_{R} TIP) 52, a right ventricular ring terminal (V_{R} RING) 54, a right ventricular shocking terminal (R_{V} COIL) 56, and an SVC shocking terminal (SVC COIL) 58, which are adapted for connection to the right ventricular tip electrode 32, right ventricular ring electrode 34, the RV coil electrode 36, and the SVC coil electrode 38, respectively.

At the core of the stimulation device 10 is a programmable microcontroller 60 which controls the various modes of stimulation therapy. As is well known in the art, the microcontroller 60 typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 60 includes the ability to process or monitor input signals (data) as controlled by a program code stored in a designated block of memory. The details of the design and operation of the microcontroller 60 are not critical to the invention. Rather, any suitable microcontroller 60 may be used that carries out the functions described herein. The use of microprocessor-based control circuits for performing timing and data analysis functions are well known in the art.

As shown in **Fig. 2**, an atrial pulse generator 70 and a ventricular pulse generator 72 generate pacing stimulation pulses for delivery by the right atrial lead 20, the right ventricular lead 30, and/or the coronary sinus lead 24 via an electrode configuration switch 74. It is understood that in order to provide stimulation therapy in each of the four chambers of the heart, the atrial and ventricular pulse generators, 70 and 72, may include dedicated, independent pulse generators, multiplexed pulse generators, or shared pulse generators. The pulse generators, 70 and 72, are controlled by the microcontroller 60 via appropriate control signals, 76 and 78, respectively, to trigger or inhibit the stimulation pulses.

The microcontroller 60 further includes timing control circuitry 79 which is used to control the timing of such stimulation pulses (e.g., pacing rate, atrio-ventricular (AV) delay, atrial interconduction (A-A) delay, or ventricular interconduction (V-V) delay, etc.) as well as to keep track of the timing of refractory periods, PVARP intervals, noise detection windows, evoked response windows, alert intervals, marker channel timing, etc., which is well known in the art.

The switch 74 includes a plurality of switches for connecting the desired electrodes to the appropriate I/O circuits, thereby providing complete electrode programmability. Accordingly, the switch 74, in response to a control signal 80 from the microcontroller 60, determines the polarity of the stimulation pulses (e.g., unipolar, bipolar, combipolar, etc.) by selectively closing the appropriate combination of switches (not shown) as is known in the art.

Atrial sensing circuits 82 and ventricular sensing circuits 84 may also be selectively coupled to the right atrial lead 20, coronary sinus lead 24, and the right ventricular lead 30, through the switch 74 for detecting the presence of cardiac activity in each of the four chambers of the heart. Accordingly, the atrial (ATR. SENSE) and ventricular (VTR. SENSE) sensing circuits, 82 and 84, may include dedicated sense amplifiers, multiplexed amplifiers, or shared amplifiers. The switch 74 determines the "sensing polarity" of the cardiac signal by selectively closing the appropriate switches, as is also known in the art. In this way, the clinician may program the sensing polarity independently of the stimulation polarity.

Each sensing circuit, 82 and 84, preferably employs one or more low power, precision amplifiers with programmable gain and/or automatic gain control, bandpass filtering, and a threshold detection circuit, as known in the art, to selectively sense the cardiac signal of interest. The automatic gain control enables the device 10 to deal effectively with the difficult problem of sensing the low amplitude signal characteristics of atrial or ventricular fibrillation. The outputs of the atrial and ventricular sensing circuits, 82 and 84, are connected to the microcontroller 60 which, in turn, are able to trigger or inhibit the atrial and ventricular pulse generators, 70 and 72, respectively, in a demand fashion in response to the absence or presence of cardiac activity in the appropriate chambers of the heart.

For arrhythmia detection, the device 10 utilizes the atrial and ventricular sensing circuits, 82 and 84, to sense cardiac signals to determine whether a rhythm is physiologic or pathologic. As used herein "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the microcontroller 60 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

Cardiac signals are also applied to the inputs of an analog-to-digital (A/D) data acquisition system 90. The data acquisition system 90 is configured to acquire intracardiac electrogram (IEGM) signals, convert the raw analog data into a digital signal, and store the digital signals for later processing and/or telemetric transmission to an external device 102. The data acquisition system 90 is coupled to the right atrial lead 20, the coronary sinus lead 24, and the right ventricular lead 30 through the switch 74 to sample cardiac signals across any pair of desired electrodes.

The microcontroller 60 is further coupled to a memory 94 by a suitable data/address bus 96, wherein the programmable operating parameters used by the microcontroller 60 are stored and modified, as required, in order to customize the operation of the stimulation device 10 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 12 within each respective tier of therapy.

Advantageously, the operating parameters of the implantable device 10 may be non-invasively programmed into the memory 94 through a telemetry circuit 100 in telemetric communication with the external device 102, such as a programmer, transtelephonic transceiver, or a diagnostic system analyzer. The telemetry circuit 100 is activated by the microcontroller by a control signal 106. The telemetry circuit 100 advantageously allows IEGMs and status information relating to the operation of the device 10 (as contained in the microcontroller 60 or memory 94) to be sent to the external device 102 through an established communication link 104.

In the preferred embodiment, the stimulation device 10 further includes a physiologic sensor 108, commonly referred to as a "rate-responsive" sensor because it is typically used to adjust pacing stimulation rate according to the exercise state of the patient. However, the physiological sensor 108 may further be used to detect changes in cardiac output, changes in the physiological condition of the heart, or diurnal changes in activity (e.g., detecting sleep and wake states). In certain embodiments, the sensor 108 includes a pressure sensor which is arranged to measure the patient's blood pressure. Accordingly, the microcontroller 60 responds by adjusting the various pacing parameters (such as rate, AV Delay, V-V Delay, etc.) at which the atrial and ventricular pulse generators, 70 and 72, generate stimulation pulses.

The stimulation device additionally includes a battery 110 which provides operating power to all of the circuits shown in **Fig. 2.** For the stimulation device 10, which employs shocking therapy, the battery 110 must be capable of operating at low current drains for long periods of time and then be capable of providing high-current pulses (for capacitor charging) when the patient requires a shock pulse. The battery 110 must also have a predictable discharge characteristic so that elective replacement time can be detected. Accordingly, the device 10 preferably employs lithium/silver vanadium oxide batteries, as is true for most (if not all) current devices.

As further shown in **Fig. 2,** the device 10 is shown as having an impedance measuring circuit 112 which is enabled by the microcontroller 60 via a control signal 114.

In the case where the stimulation device 10 is intended to operate as an implantable cardioverter/ defibrillator (ICD) device, it must detect the occurrence of an arrhythmia, and automatically apply an appropriate electrical shock therapy to the heart aimed at terminating the detected arrhythmia. To this end, the microcontroller 60 further controls a shocking circuit 116 by way of a control signal 118. The shocking circuit 116 generates shocking pulses of low (up to 0.5 Joules), moderate (0.5 - 10 Joules), or high energy (11 to 40 Joules), as controlled by the microcontroller 60. Such shocking pulses are applied to the patient's heart 12 through at least two shocking electrodes, and as shown in this embodiment, selected from the left atrial coil electrode 28, the RV coil electrode 36, and/or the SVC coil electrode 38. As noted above, the housing 40 may act as an active electrode in combination with the RV electrode 36, or as part of a split electrical vector using the SVC coil electrode 38 or the left atrial coil electrode 28 (i.e., using the RV electrode as a common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 Joules), delivered asynchronously (since R-waves may be too disorganized), and pertaining exclusively to the treatment of fibrillation. Accordingly, the microcontroller 60 is capable of controlling the synchronous or asynchronous delivery of the shocking pulses.

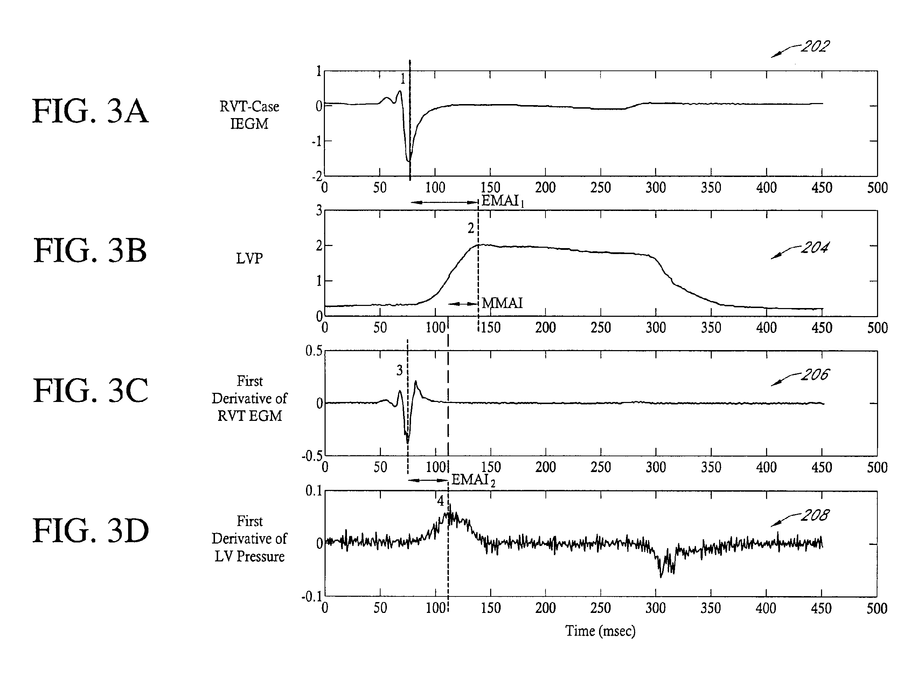

Figs. 3A and 3B show exemplary wave forms of observed electrical 202 and 204 mechanical activity of the patient's heart 12 provided by one embodiment of the device 10. More particularly, Fig. 3A illustrates a waveform corresponding to an intracardiac electrogram (IEGM) and which, in this particular embodiment, corresponds to electrical signals observed between the right ventricular tip electrode 32 and the case or can 40. In other embodiments, other IEGM configurations employing either unipolar and/or bipolar sensing arrangements of other electrodes can also be utilized to measure electrical activity of the heart 12. Fig. 3A shows the time-varying nature of this electrical activity 202, (in this embodiment over one cardiac cycle) including the atrial and ventricular activity. In this embodiment, the microcontroller 60 has designated a first monument 1 corresponding in this particular embodiment to the peak observed electrical activity of an R-wave. In this embodiment, the first monument 1 would be characterized by the microcontroller 60 to include both the peak observed magnitude of the electrical activity 202 as well as a time stamp or marker corresponding to the timing of this observed peak.

**Fig. 3B** illustrates an exemplary wave form of observed mechanical activity 204 of the patient's heart 12. In this particular embodiment, **Fig. 3B** illustrates the output of a physiologic sensor 108 configured as a pressure sensor. The physiologic sensor 108 is arranged to monitor the output pressure of the patient's left ventricle and again **Fig. 3B** illustrates the variation of this pressure signal corresponding to the mechanical activity 204 over time. One example of a suitable physiologic sensor 108 is the micro-electromechanical systems (MEMS) based implantable capacitive pressure sensors from Integrated Sensing Systems, Inc. (ISSYS) of Ypsilanti, Michigan.

In other embodiments, the sensor 108 comprises an accelerometer arranged to provide signals indicative of the timing and intensity of mechanical activity/movement of the heart 12. In yet other embodiments, the sensor 108 is configured to evaluate a transthoracic impedance of the patient. The device 10 monitors the mechanical activity/movement of the heart 12 by evaluating artifacts of the time-varying transthoracic impedance arising from the heart's mechanical activity which in certain embodiments includes appropriate signal processing to isolate the heart motion artifacts. In further embodiments, the device 10 analyzes the left atrial signals corresponding to the LA depolarizations to monitor the mechanical activity of the heart 12. Thus it will be appreciated that **Fig. 3B** illustrates simply one particular embodiment of monitoring the mechanical activity/movement of the patient's heart 12 and that a number of different procedures and components can be employed in particular applications.

The microprocessor 60 evaluates the mechanical activity 204 and designates a second monument 2 corresponding in this embodiment to a peak in the observed mechanical activity 204. Again, the second monument 2 would also comprise information corresponding to the observed peak magnitude/intensity of the observed mechanical activity 204 as well as to the relative timing of the occurrence of this peak.

Thus, the microprocessor 60 can compare the relative timing of the first monument 1 and the second monument 2 to determine a delay or activation interval between the observed electrical activity 202 and mechanical activity 204. It will be further appreciated that monuments other than a peak amplitude, such as a zero crossing, peak rate of change, inflection point, etc. can be established for electrical and mechanical activity of the heart 12 in other embodiments.

For example, **Figs. 3C** and **3D** illustrate a further embodiment wherein the signals indicative of the electrical activity 202 and mechanical activity 204 are processed to obtain derived indicators of the electrical activity and mechanical activity 206, 208, respectively. In this particular embodiment, the derived indicator of electrical characteristics and mechanical characteristics 206, 208, respectively, comprise the first derivative of the measured indicators of the electrical activity 202 and the mechanical activity 204, respectively. The first derivatives can be obtained via hardware processing of measured signals, such as with an operational amplifier (op-amp) differentiator circuit, as well as with appropriate software processing of the measured signals.

in the embodiments illustrated by **Figs.** 3C and **3D,** the derived indicators 206, 208 comprising the first derivatives of the base signals corresponding to observed electrical activity 202 and mechanical activity 204 provide alternative indicators of the morphology of the underlying electrical and mechanical activity 202, 204. More particularly, the first derivatives of signals indicative of these processes provide indicators directly proportional to the time rate of change of these physiological processes which in certain embodiments are more readily processed and evaluated, such as by the microcontroller 60, to establish monuments.

As can be seen in a comparison of **Figs. 3A** and **3C,** a third monument 3 is designated for the derived indicator of electrical characteristics 206 which corresponds to the relatively sharp local maxima of the peak of the R-wave. Similarly, a comparison of the waveforms of **Figs. 3B** and **3D** show that the fourth monument 4 indicated on Fig. 3D corresponds to the peak rate of change of the mechanical activity 204 or generally the steepest slope of the waveform illustrated for the mechanical activity 204 in Fig. **3B.** Thus, the fourth monument 4 corresponds generally to the peak mechanical output period in this cardiac cycle where the peak cardiac output corresponds generally to the largest rate of increase in the pressure generated by the left ventricle as indicated by the physiologic sensor 108 arranged to measure this pressure.

Thus, depending upon the indications for a particular application, these embodiments provide signals and indicators providing a wide variety of information relating to the electrical activity 202 and mechanical activity 204 as well as indicators derived therefrom, such as the derived indicators 206, 208. For example, in one embodiment, the second monument 2 can be designated to correspond to the local peak pressure and the fourth monument 4 can be designated to correspond generally to the peak rise in pressure which would not generally occur at the same time as the local peak pressure but would rather precede it. Thus, the device 10 including the microcontroller 60 can evaluate the relative timing between indicators of the electrical activity 202 as well as indicators derived therefrom 206 as well as the indicators of the mechanical activity 204 and derived indicators 208 arising from the stimulation indicated by the electrical activity 202, 206 and monuments thereof, such as the second monument 2 corresponding to peak pressure and the fourth monument 4 corresponding to peak rate of pressure change. The device 10 can thus analyze these indicators of electrical activity 202, 206 and mechanical activity 204, 208 to evaluate the relative correspondence therebetween to evaluate the patient's condition, such as the progression of an HF condition.

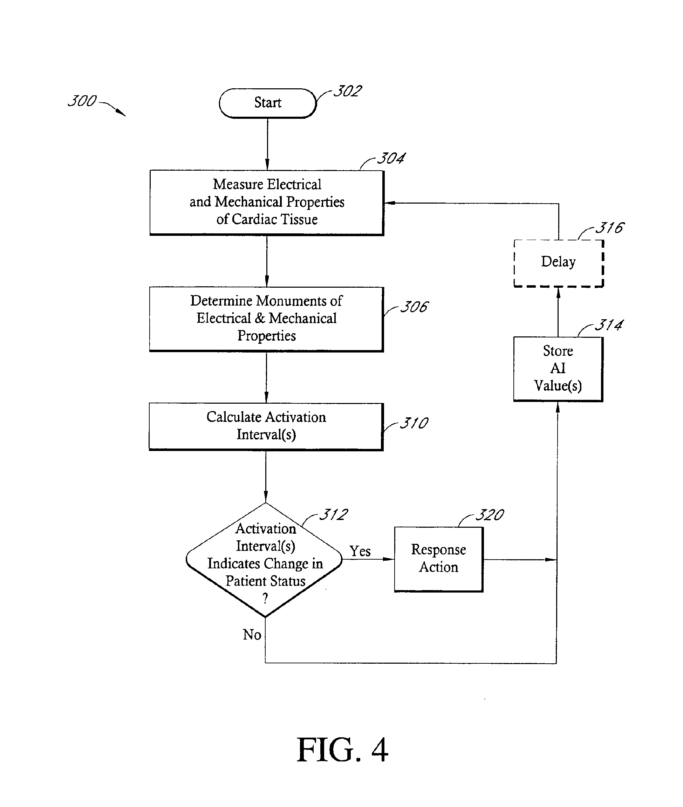

**Fig. 4** illustrates a flow chart corresponding to one embodiment of a method 300 of evaluating the electrical ― mechanical activity of the heart 12. Beginning from a start state 302, the method comprises a state 304 wherein both the electrical and mechanical properties of the heart 12 are measured, such as illustrated in **Figs. 3A** and **3B**. A state 306 follows wherein monuments of the electrical and mechanical activity is determined. In various embodiments, this can include the monuments 1 and 2 corresponding to the direct measurements of the electrical activity 202 and mechanical activity 204 and in other embodiments can include in addition or as an alternative to these, the monuments 3 and 4, corresponding to derived indicators of the electrical characteristics and mechanical characteristics 206, 208, respectively.

This is followed by a state 310 wherein calculations are performed to determine one or more activation intervals between selected monuments of the observed electrical and mechanical activity 202, 204, 206, 208. One embodiment of an activation interval comprises a first electro-mechanical activation interval (EMAI₁) illustrated with respect to **Figs. 3A** and **3B** as the delay or interval between the peak of the electrical activity 202 and the corresponding mechanical activity 204. The EMAI₁ is an indicator of the delay between the electrical activity 202 triggering a heart contraction and the corresponding mechanical activity 204 where the second monument 2 indicates the peak pressure generated, in this embodiment as measured at the left ventricle.

Another embodiment of an activation interval comprises a second electro-mechanical activation interval (EMAI₂) corresponding to the delay or interval between the third monument 3 and the fourth monument 4. The EMAI₂ corresponds generally to the delay or interval between the peak of the change of the patient's R-wave and the timing of peak pressure change indicated by the fourth monument 4 corresponding generally to the period of maximal ventricular effort which would typically precede the actual peak generated pressure. In other embodiments, the interval between the first monument 1 of the observed electrical activity 202 and the fourth monument 4 of the derived indicator of mechanical activity 208 define an EMAI. Yet another embodiment defines an EMAI as the interval between the third monument 3 of the derived indicator of electrical activity 206 and the second monument 2 of the observed mechanical activity 204.

Another embodiment of an activation interval comprises a mechanical activation interval (MMAI), in this embodiment the interval or delay between the peak rate of change in ventricular pressure indicated by the fourth monument 4 and the peak generated pressure indicated by the second monument 2. The MMAI indicates the relative timing or delay between the peak muscular effort of the ventricles and the period at which the peak pressure is subsequently generated. Following the calculations in state 310 of one or more of the activation intervals, a state 312 follows wherein these activation intervals are evaluated for further determination.

State 312 generally determines in this embodiment whether the calculation of the one or more activation intervals indicates a change in the patient's status. For most applications, it would be expected that each activation interval, such as the EMAI₁, EMAI₂ and MMAI would have a positive value and the nominal ranges of these activation intervals for a given patient would be readily determined by a clinician or other person of ordinary skill in the art.

The determination of state 312 is based at least in part on the assumption that a marked elongation or extension of one or more of these activation intervals would be indicative of an increasing disassociation between the electrical activity and mechanical activity 204. Thus, in one embodiment, a determination of an elongated current EMAI₁ as compared to one or more previously determined EMAI₁ and/or an increase in the current determined EMAI₁ in excess of a determined threshold, would indicate that an increased decoupling of the electrical and mechanical activity of the heart 12 is occurring. In certain embodiments, this increased decoupling/disassociation would be indicative either of an onset of an HF condition or the worsening of an existing HF condition. Similarly, an elongation of the MMAI as compared to previously observed MMAIs or a determination of an MMAI in excess of a determined threshold value similarly indicate a degradation in the contractual capability of the heart 12 which also is indicative of an onset or worsening of an HF condition.

In other embodiments, a change to a longer activation interval and/or initial determination of a markedly elongated activation interval is indicative that a cardiac resynchronization therapy (CRT) regimen is not having the desired effect. Conversely, a reduction of the activation interval and/or observation of one or more activation intervals within a corresponding threshold is a positive indicator for the efficacy of the CRT. Thus, measurement and analysis of one or more activation intervals can be utilized in certain embodiments as determinants for adjustment, continuation, initiation, or cessation of particular therapies, such as CRT, provided by the device 10.

In yet other embodiments, the measured and calculated activation intervals can be evaluated as feedback indicators for programming/implantation parameters of the device 10. In one embodiment, the activation intervals can be evaluated with different programming settings of parameters affecting the AV/VV timing. The activation intervals would be expected in many applications to vary with adjustments to the programming of these timing parameters and the varying activation intervals can be used to improve the adjustment of the programmed parameters for improved therapy delivery. In yet other embodiments, the activation intervals would likewise be expected to vary depending on lead placement, for example the placement of the left ventricular lead. The varying activation intervals are evaluated, such as in an electrophysiology (EP) catheter lab, during lead placement and are used as clinical tools to select the placement location(s) of one or more leads for improved sensing and delivery of therapy by the device 10 for a particular patient.

The selection of an appropriate activation interval, such as one or more of the EMAI₁, EMAI₂, and/or MMAI as well as the designation of appropriate monuments for determination of these activation intervals, would be provided in certain embodiments in a user-selectable manner such that a skilled clinician familiar with the characteristics of the device 10 as well as the condition of the particular patient, could determine appropriate monuments as well as select appropriate activation intervals and threshold or limit values for appropriate determination of the state 312 of a change in the patient's status of interest.

Thus, if the determination of state 312 is negative, e.g., that no significant change in the patient's condition has been observed, data such as the calculations of one or more of the activation intervals of state 310 can be stored in state 314. This stored data can be accessed, such as via a telemetric link 104, for further evaluation by a clinician. In certain embodiments, a programmable delay of state 316 would occur wherein a delay period occurs before repetition of the previously described steps of the method 300 would occur. Thus, the delay state 316 can be provided in certain embodiments such that the method 300 is iteratively performed at certain intervals, such as weekly, daily, etc.

If the determination of state 312 is affirmative, e.g., that a change in the patient's status has been observed, a state 320 would occur wherein an appropriate response action would be taken. In certain embodiments, the action of state 320 comprises enablement or activation of one or more features of the implantable device 10. Thus, for example detection of an onset of an HF condition or a worsening of the same can indicate a change in the operation of the therapy delivered by the device 10 and/or a change in the programmed parameters for determination of delivery of therapy by the device 10. In other embodiments, the action of state 320 comprises activation of a flag or other alert, such as via the telemetric link 104, to alert the patient and/or attending clinical personnel of the detection of the change in the patient's condition from state 312. Of course, an alert or flag set in state 320 can correspond to a positive indicator, for example indicating positive effect of a CRT regimen, as well as a negative indicator, for example indicating different lead placement. In yet other embodiments, the action of state 320 comprises simply storing the data relating to the detection of change in patient status from state 312 in the data storage state 314 such that the data could be accessed at a later time. Thus, in certain embodiments, the response action taken in state 320 is performed by the device 10 itself, in certain embodiments the action of state 320 is performed by attending clinical personnel, and in yet other embodiments the action of state 320 involves actions taken both by the device 10 and by external personnel and/or one or more external devices 102 in communication with the device 10.

Thus, the device 10 and method 300 provide an effective relatively inexpensive capability for evaluating the patient's status on a long-term basis, such as to monitor a degree of heart failure. This evaluation can be performed frequently, such as weekly, daily, etc. but in a manner that each incidence of evaluation does not require the immediate presence and input of a skilled clinician nor the use of expensive, relatively complex diagnostic equipment. The device 10 can be provided with the additional functionality of the method 300 with the incremental expense of revision to the operating software of the device 10 as well as appropriate designation of parameters and thresholds by the attending clinician. The device 10 and method 300 provide the further advantage of the ability to immediately notify, such as via a telemetric link 104, should any change in the patient's status indicate immediate intervention and alternatively, can store data indicative of the patient's status for subsequent analysis and possible reprogramming or alteration in therapy should the change in an status indicate a less urgent need.

## Claims

1. An implantable medical device (10) comprising: an electrical activity detector (82,84) for detecting electrical activity of a patient's heart (12); and a mechanical activity detector (108) for detecting mechanical activity of the heart (12); **characterised by** means for determining a delay between corresponding monuments of the electrical activity and of the mechanical output for a given cardiac cycle; and means for evaluating the patient's condition based at least partially on the delay.

2. A device as claimed in Claim 1, **characterised in that** the means for evaluating the patient's condition is arranged to evaluate the efficacy of a previously instituted cardiac resynchronization therapy (CRT) regimen.

3. A device as claimed in Claim 1 or Claim 2, **characterised by** means for providing therapy according to at least one of a programmable atrioventricular (AV) delay and a programmable ventricular-ventricular (VV) delay, and means for programming at least one of the AV and VV delays of the device based at least in part on the evaluation of the patient's condition.

4. A device as claimed in any preceding Claim, **characterised in that** the means for detecting mechanical activity (108) is arranged to measure the pressure of blood pumped from the heart (12).

5. A device as claimed in any preceding Claim, **characterised in that** the means for evaluating the patient's condition is arranged to evaluate the degree of heart failure.

6. A device as claimed in any preceding Claim, **characterised by** means for generating an alert based on the evaluation of the patient's condition.

7. A device as claimed in any preceding Claim, **characterised by** means for communicating (100) results of the evaluation to an external device (102).

8. A device as claimed in any preceding Claim, **characterised in that** the implantable device is capable of delivering therapy to the patient and includes means for adjusting one or more therapy-related parameters of the device based at least in part on the evaluating.

9. An implantable medical device (10) comprising at least one implantable sensing electrode (42-58) configured for measuring electrical activity of a patient's heart; **characterised by**: at least one implantable mechanical sensor (108) configured to measure the mechanical activity of the heart; and a controller (60) in communication with the sensing electrode (42-58) and mechanical sensor (108), wherein the controller (60) is operative to evaluate the relative timing between the electrical and mechanical activity and determine a health indicator based at least in part on the evaluation.

10. A device as claimed in Claim 9, **characterised in that** the controller evaluates the relative timing between the electrical and mechanical activity by comparing a delay between corresponding peaks of the electrical and of the mechanical activity against a threshold value.

11. A device as claimed in Claim 9, or Claim 10, **characterised in that** the controller determines monuments of each of the electrical and mechanical activity and evaluates the relative timing as an interval between corresponding monuments of the electrical and mechanical activity.

12. A device as claimed in any of Claims 9 to 11, **characterised in that** the health indicator is indicative of progression of a heart failure condition and wherein a worsening condition is indicated by an elongation of an activation interval between the electrical and mechanical activity.

13. A device as claimed in any of Claims 9 to 12, **characterised in that** it further comprises memory and wherein the controller (60) stores data corresponding to the evaluation and determination of the health indicia.

14. A device as claimed in any of Claims 9 to 13, **characterised in that** it further comprises a stimulation generator (70) and at least one stimulation electrode (42,46,48) connected to the stimulation generator (70) and configured for delivery of therapeutic stimulation to the patient, and the controller (60) induces delivery of the stimulation based at least partially on the evaluation of the signals from the sensing electrode (42-58) and mechanical sensor (108).

15. A device as claimed in any of Claims 9 to 14, **characterised in that** it further comprises a telemetry circuit (100) and the device can telemetrically communicate data corresponding to the evaluation and determination of the health indicia to an external device (102).
